# EUROPEAN PATENT APPLICATION

(11) **EP 4 834 296 A1**
(43) Date of publication of application: **04.11.2026**
(21) Application number: 24913515.3
(22) Date of filing: 23.12.2024
(51) Int. Cl.: A61F 7/00

(54) **WET THERMOTHERAPY DEVICE CAPABLE OF ALLEVIATING NASAL DISCHARGE AND NASAL CONGESTION SYMPTOMS BY USING CONTROL OF TEMPERATURE, FLOW RATE AND PRESSURE OF STEAM**

(30) Priority: 26.12.2023 KR 20230191194
(71) Applicant: Rhinocare Korea Inc., Gumi-si, Gyeongsangbuk-do 39371 (KR); Rhinocare Ltd, Ra'anana (IL)
(72) Inventor: HWANG, Jae Hoon, Seoul 05504 (KR)
(74) Representative: Pfenning, Meinig & Partner mbB
(86) International application number: PCT/KR2024/020970
(87) International publication number: WO 2025/143722

(57) **Abstract**

The present invention relates to a wet thermotherapy device capable of alleviating nasal discharge and nasal congestion symptoms by using the control of the temperature, flow rate and pressure of steam, comprising: a steamer having an opened upper portion so as to accommodate water therein, and then heat the water such that steam is generated; a cover which closes the opened upper portion of the steamer so as to prevent water leakage, and which has a discharge hole provided in the center thereof so that the steam can be discharged to the outside; a nozzle having a tube formed at one end thereof such that the tube is coupled to the discharge hole so as to receive the steam, and having a plurality of injection holes provided at the other end thereof such that the steam can be injected into each of the nostrils; and a main body which is formed such that the steamer can be mounted on the upper portion thereof, and which controls the operation of the steamer and supplies external air into the steamer so as to control that the temperature, pressure and flow rate of the steam injected from the nozzle are constantly maintained.

## Description

### TECHNICAL FIELD

The disclosure relates to a wet thermotherapy device capable of improving runny nose and nasal congestion symptoms using control of temperature, flow rate, and pressure of steam, and more particularly, to a wet thermotherapy device capable of improving runny nose and nasal congestion symptoms using control of temperature, flow rate, and pressure of steam, which sprays steam generated by boiling water into the inside of the nasal cavity to treat inflammation, runny nose, and nasal congestion inside the nasal cavity and to prevent respiratory diseases.

### BACKGROUND ART

A conventional rhinitis treatment device of a simple thermal treatment type simply heats only air to a certain temperature and injects the air into the nose. In this type, since humidity is low, the nasal cavity becomes dry and stress is applied to the nasal mucosa inside the nose, causing side effects that interfere with normal nasal mucosa activity.

In addition, a conventional portable rhinitis treatment device of a light irradiation type also fails to maintain sufficient humidity, and due to the anatomical structure of the paranasal sinus, light cannot be irradiated to the inflamed area, making it difficult to perform proper treatment.

Further, in the case of a simple steamer type and an ultrasonic humidification type rhinitis treatment device, although humidity may be maintained, the temperature of steam cannot be maintained constant, and high-temperature steam cannot reach the inner inflamed area due to insufficient pressure and flow rate, so an accurate thermal treatment effect cannot be expected.

Further, in the conventional portable insertion-type rhinitis treatment device of a light irradiation type, the inserted portion physically directly contacts the epidermis-mucosa inside the nose for a long time, which may cause side effects of irritating the nasal mucosa and damaging mucosal cells, and may cause a risk of secondary infection due to contact inflammation.

In addition, since the inflamed area is swollen, light cannot reach the entire inflamed area evenly due to the structure, and the temperature of the nasal cavity cannot be maintained at 43 degrees, so accurate treatment of inflammation cannot be performed by light irradiation alone.

On the other hand, when a conventional liquid spray-type medication device is used, although a temporary therapeutic effect may be obtained, there is a concern that drug resistance and side effects may occur depending on the person when used for a long term, and fundamental treatment is difficult with drug therapy alone.

In addition, in the case of a conventional method of treatment by spraying gas such as carbon dioxide, the inside of the nose may become dry, which may lead to undesirable results.

Desirable conditions for local thermal treatment of rhinitis are that the temperature in the nasal cavity should be maintained at a specific treatable temperature for a certain period of time, and high-temperature steam should be continuously supplied and maintained to the inflamed area of the paranasal sinus while overcoming the anatomical resistance of the nasal cavity. However, since the conventional techniques described above do not satisfy these conditions, there is a problem that the therapeutic effect is reduced.

### DETAILED DESCRIPTION OF THE INVENTION

### TECHNICAL PROBLEM

An object of the disclosure for solving the above problems is to provide a wet thermotherapy device capable of improving runny nose and nasal congestion symptoms using control of temperature, flow rate, and pressure of steam, in which the temperature of steam is maintained constant and the flow rate and pressure of steam are maintained constant so that the steam may be delivered to the inside of the nasal cavity in order to maximize the effect of the local thermal treatment for rhinitis.

Further, another object of the disclosure is to provide a wet thermotherapy device capable of improving runny nose and nasal congestion symptoms using control of temperature, flow rate, and pressure of steam, which is harmless to the human body even when inhaled by a user or used for a long time by using only saturated steam generated by purely boiling water without using drugs or other gases and light rays.

Further, another object of the disclosure is to provide a wet thermotherapy device capable of improving runny nose and nasal congestion symptoms using control of temperature, flow rate, and pressure of steam, which may remove harmful germs without unnecessary irritation to the affected area, improve runny nose symptoms and nasal congestion symptoms, alleviate inflammation, and prevent respiratory diseases by spraying steam without physical contact with the affected area for treatment.

Further, another object of the disclosure is to provide a wet thermotherapy device capable of improving runny nose and nasal congestion symptoms using control of temperature, flow rate, and pressure of steam, which is capable of customized treatment according to a user's situation by recommending a user's treatment condition based on external environment information and optimizing operating conditions.

### TECHNICAL SOLUTION

A wet thermotherapy device capable of improving runny nose and nasal congestion symptoms using control of temperature, flow rate, and pressure of steam according to the disclosure for solving the above problems includes: a steamer having an open upper portion to accommodate water therein and heat the water to form steam; a cover provided with a discharge hole in the center to seal the open upper portion of the steamer so as to prevent water from leaking and to discharge steam to the outside; a nozzle in which a tube is formed at one end such that the tube is coupled to the discharge hole to receive steam, and a plurality of injection holes are provided at the other end so that steam may be sprayed to each nostril; and a main body which is formed such that the steamer may be mounted on an upper portion thereof and which controls the operation of the steamer and supplies external air into the steamer so as to maintain the temperature, pressure, and flow rate of steam sprayed from the nozzle constant.

Further, in the wet thermotherapy device capable of improving runny nose and nasal congestion symptoms using control of temperature, flow rate, and pressure of steam according to the disclosure, the main body includes: a blower fan that forms external air with a set flow rate and pressure by rotation and supplies the external air to the steamer; a filter formed on a front surface of the blower fan to collect and purify foreign matter, germs, and viruses mixed in the external air; an outside air temperature sensor formed on the front surface of the blower fan to measure a temperature of the external air being sucked in so as to adjust in advance the temperature of steam generated in the steamer; and a supply flow path for supplying the external air supplied by the blower fan into the steamer, wherein the blower fan maintains the flow rate of the external air at 2.0 to 2.5 CFM and the pressure at 80 to 85 mmH2O.

Further, in the wet thermotherapy device capable of improving runny nose and nasal congestion symptoms using control of temperature, flow rate, and pressure of steam according to the disclosure, the steamer includes: a heating container having an open upper portion to accommodate water and made of a stainless steel material; a heater formed on a lower surface of the heating container to receive power from the main body and heat the heating container to generate steam; an inlet flow path formed on one upper side of the heating container to allow external air supplied from the main body to flow into an upper portion of the heating container; and an inside air temperature sensor formed on the other upper side of the heating container to measure a temperature of steam in a state in which external air is introduced and to control the temperature of the heater, wherein the heater maintains the temperature of steam discharged through the nozzle at 40 to 45 degrees Celsius.

Further, in the wet thermotherapy device capable of improving runny nose and nasal congestion symptoms using control of temperature, flow rate, and pressure of steam according to the disclosure, the main body is formed to wirelessly communicate with a smartphone in which information on a user's treatment mode, treatment time, treatment cycle, and treatment history is input, such that the operation time of the steamer and the temperature, flow rate, and pressure of steam are automatically adjusted by the smartphone.

Further, in the wet thermotherapy device capable of improving runny nose and nasal congestion symptoms using control of temperature, flow rate, and pressure of steam according to the disclosure, the main body receives at least one of weather information, fine dust information, health information, disease information, and prescription information of a current region through wireless communication and recommends a recommended treatment mode according to an environmental condition or a user condition.

Further, in the wet thermotherapy device capable of improving runny nose and nasal congestion symptoms using control of temperature, flow rate, and pressure of steam according to the disclosure, the nozzle further includes an adjustment cap formed on an outer surface thereof and formed to adjust an interval of the plurality of injection holes to a user's nostril position by rotation.

### ADVANTAGEOUS EFFECTS

As described above, according to the wet thermotherapy device capable of improving runny nose and nasal congestion symptoms using control of temperature, flow rate, and pressure of steam according to the disclosure, there is an effect that the temperature of steam is maintained constant and the flow rate and pressure of steam are maintained constant so that the steam may be delivered to the inside of the nasal cavity in order to maximize the effect of the local thermal treatment for rhinitis.

Further, according to the wet thermotherapy device capable of improving runny nose and nasal congestion symptoms using control of temperature, flow rate, and pressure of steam according to the disclosure, there is an effect that it is harmless to the human body even when inhaled by a user or used for a long time by using only saturated steam generated by purely boiling water without using drugs or other gases and light rays.

Further, according to the wet thermotherapy device capable of improving runny nose and nasal congestion symptoms using control of temperature, flow rate, and pressure of steam according to the disclosure, there is an effect that harmful germs may be removed without unnecessary irritation to the affected area, runny nose symptoms and nasal congestion symptoms may be improved, inflammation may be alleviated, and respiratory diseases may be prevented by spraying steam without physical contact with the affected area for treatment.

Further, according to the wet thermotherapy device capable of improving runny nose and nasal congestion symptoms using control of temperature, flow rate, and pressure of steam according to the disclosure, there is an effect that customized treatment according to a user's situation is possible by recommending a user's treatment condition based on external environment information and optimizing operating conditions.

### BRIEF DESCRIPTION OF THE DRAWINGS

FIG. 1 is a perspective view illustrating an external appearance of a wet thermotherapy device capable of improving runny nose and nasal congestion symptoms using control of temperature, flow rate, and pressure of steam according to the disclosure.
FIG. 2 is an assembly view illustrating a state in which a steamer of the wet thermotherapy device capable of improving runny nose and nasal congestion symptoms using control of temperature, flow rate, and pressure of steam according to the disclosure is separated.
FIG. 3 is a perspective view illustrating a coupling portion between a main body and a steamer of the wet thermotherapy device capable of improving runny nose and nasal congestion symptoms using control of temperature, flow rate, and pressure of steam according to the disclosure.
FIG. 4 is a cross-sectional view illustrating an internal structure of the steamer of the wet thermotherapy device capable of improving runny nose and nasal congestion symptoms using control of temperature, flow rate, and pressure of steam according to the disclosure.
FIG. 5 is a configuration view illustrating a coupling and operation structure of the wet thermotherapy device capable of improving runny nose and nasal congestion symptoms using control of temperature, flow rate, and pressure of steam according to the disclosure.
FIG. 6 is a perspective view illustrating a variable nozzle of the wet thermotherapy device capable of improving runny nose and nasal congestion symptoms using control of temperature, flow rate, and pressure of steam according to the disclosure.
FIG. 7 is a front view illustrating a state in which a position where steam is discharged is varied by an adjustment cap in the nozzle of the wet thermotherapy device capable of improving runny nose and nasal congestion symptoms using control of temperature, flow rate, and pressure of steam according to the disclosure.
FIG. 8 is an exemplary view illustrating a state in which the wet thermotherapy device capable of improving runny nose and nasal congestion symptoms using control of temperature, flow rate, and pressure of steam according to the disclosure is linked with a smartphone.
FIG. 9 is a flowchart illustrating a control process logic of the wet thermotherapy device capable of improving runny nose and nasal congestion symptoms using control of temperature, flow rate, and pressure of steam according to the disclosure.

100: main body
101: coupling groove
110: power supply unit
120: control unit
130: display
140: communication module
150: outside air temperature sensor
160: filter
170: blower fan
180: supply flow path
190: first connector
200: steamer
201: coupling hook
210: heater
220: heating container
230: inside air temperature sensor
240: cover
241: discharge hole
250: tube
260: nozzle
261: injection hole
262: adjustment cap
270: second connector
280: inlet flow path
300: smartphone
400: Internet network

### BEST MODE FOR CARRYING OUT THE INVENTION

Specific features and advantages of the disclosure will be described in detail below with reference to the accompanying drawings. Prior to this, when it is determined that a detailed description of functions and configurations related to the disclosure may unnecessarily obscure the gist of the disclosure, the detailed description will be omitted.

The disclosure relates to a wet thermotherapy device capable of improving runny nose and nasal congestion symptoms using control of temperature, flow rate, and pressure of steam, and more particularly, to a wet thermotherapy device capable of improving runny nose and nasal congestion symptoms using control of temperature, flow rate, and pressure of steam, which sprays steam generated by boiling water into the inside of the nasal cavity to treat inflammation and nasal congestion inside the nasal cavity and prevent respiratory diseases.

Hereinafter, preferred embodiments of the disclosure will be described in detail with reference to the accompanying drawings.

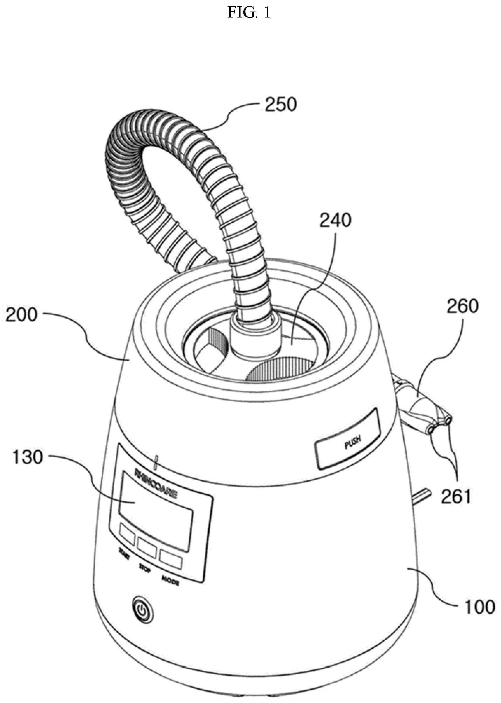

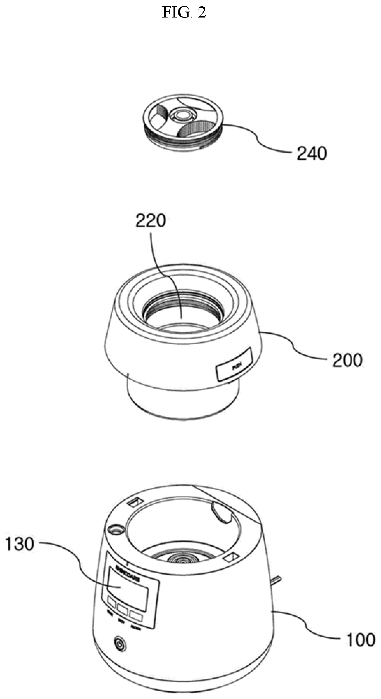

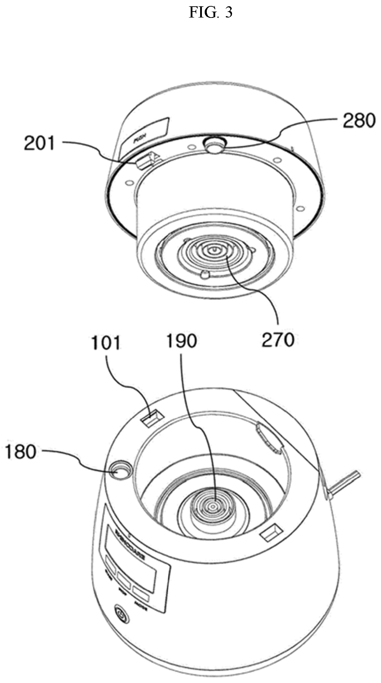

FIG. 1 is a perspective view illustrating an external appearance of a wet thermotherapy device capable of improving runny nose and nasal congestion symptoms using control of temperature, flow rate, and pressure of steam according to the disclosure, FIG. 2 is an assembly view illustrating a state in which a steamer of the wet thermotherapy device capable of improving runny nose and nasal congestion symptoms using control of temperature, flow rate, and pressure of steam according to the disclosure is separated, and FIG. 3 is a perspective view illustrating a coupling portion between a main body and a steamer of the wet thermotherapy device capable of improving runny nose and nasal congestion symptoms using control of temperature, flow rate, and pressure of steam according to the disclosure.

As illustrated in FIGS. 1 to 3, the wet thermotherapy device capable of improving runny nose and nasal congestion symptoms using control of temperature, flow rate, and pressure of steam according to the disclosure includes a steamer 200 having an open upper portion to accommodate water therein and heat the water to form steam, a cover 240 provided with a discharge hole 241 in the center to seal the open upper portion of the steamer 200 so as to prevent water from leaking and to discharge steam to the outside, a nozzle 260 in which a tube 250 is formed at one end such that the tube 250 is coupled to the discharge hole 241 to receive steam, and a plurality of injection holes 261 are provided at the other end so that steam may be sprayed to each nostril, and a main body 100 which is formed such that the steamer 200 may be mounted on an upper portion thereof and which controls the operation of the steamer 200 and supplies external air into the steamer 200 so as to maintain the temperature, pressure, and flow rate of steam sprayed from the nozzle 260 constant.

The steamer 200 is formed such that an upper center thereof is open so that water may be filled and accommodated therein, and when inserted into the main body 100, the steamer 200 is supplied with power from the main body 100 and is controlled to heat and evaporate water accommodated therein, and is used to generate steam.

In this case, the steamer 200 is formed in a T-shape in which the diameters of the upper end and the lower end are different from each other, and the lower end is formed to have a smaller diameter than the upper end so as to be inserted into the main body 100, and the upper end is formed to be mounted and fixed on an upper surface of the main body 100.

Since the steamer 200 is formed in a structure detachable from the main body 100, the steamer 200 may be carried in a state separated from the main body 100, so that water may be filled therein or water accommodated therein may be emptied.

The cover 240 is used to seal the open upper portion of the steamer 200 to prevent water from leaking to the outside when the steamer 200 is carried and moved, and to prevent external air from being introduced, thereby maintaining the internal pressure and temperature of the steamer 200 constant.

Further, the discharge hole 241 is provided in the center of the cover 240 so that steam may be discharged, and the tube 250 formed in the nozzle 260 is coupled thereto so that the steam may be controlled to be discharged only through the nozzle 260.

In order for the cover 240 to be coupled to the steamer 200, a thread is formed on an outer circumferential surface of the cover 240, and a thread is also formed at an upper end of the open portion of the steamer 200, so that the cover 240 and the steamer 200 may be fastened and fixed by the threads.

Further, it is preferable that a sealing is formed on the upper outer circumferential surface of the cover 240 to prevent water or steam from leaking through a gap between the threads.

The nozzle 260 includes the tube 250 made of a soft material at one end so as to receive steam generated from the steamer 200, and two injection holes 261 are provided at the other end so as to spray to the nostrils of the user.

The tube 250 is formed to be coupled to the discharge hole 241 formed in the center of the cover 240, and is used to move steam generated in the steamer 200 to the nozzle 260. Since the tube 250 is formed of a flexible material, the shape thereof may be varied so that the nozzle 260 may be positioned at the nose of the user.

Further, in order to prevent steam from leaking through the gap between the discharge hole 241 and the tube 250 when the tube 250 is coupled to the discharge hole 241, it is preferable that the outer surface of the discharge hole 241 is coupled in a form of being inserted into the tube 250.

The injection holes 261 formed in the nozzle 260 are formed such that the steam passing through the tube 250 is respectively sprayed to each nostril and penetrates into the nasal cavity in accordance with the user's nostril position, and the user may treat rhinitis by spraying the steam through the nostril into the nasal cavity by allowing the injection holes 261 to face the front of the nostrils while gripping the nozzle 260.

The main body 100 is formed such that an upper portion thereof is open so that a lower end of the steamer 200 may be inserted, and is used to receive power from the outside, supply power for generating steam in the steamer 200, and control the steamer 200.

Further, the main body 100 may control the pressure and flow rate by sucking in external air and supplying it into the steamer 200 so that the steam generated inside the steamer 200 is mixed with the external air supplied by the main body 100 and is sprayed through the nozzle 260 in a state having a constant pressure and flow rate.

That is, since it is difficult to penetrate into the nasal cavity only with the power of steam generated by heating water, the external air supplied from the main body 100 is mixed with the steam while maintaining a set flow rate and pressure and is discharged through the nozzle 260, so that the steam penetrates into the nasal cavity and the therapeutic effect may be increased.

To this end, a display 130 for operating control and setting change is formed on an outer surface of the main body 100, and a current setting value and operating state may be identified through the display 130, and the setting value may be changed and controlled using buttons formed around the display 130.

In order for the external air supplied from the main body 100 to be supplied into the steamer 200, a supply flow path 180 is formed inside the main body 100 and is formed to be discharged to the upper portion of the main body 100, and an inlet flow path 280 for being connected to the supply flow path 180 is provided on a lower surface of an upper end of the steamer 200.

That is, when the steamer 200 is coupled to the main body 100, the inlet flow path 280 may be inserted into the supply flow path 180 to be connected to each other, and external air sucked in and supplied from the main body 100 may be supplied into the steamer 200 and discharged through the nozzle 260.

Further, in order to prevent the steamer 200 from being arbitrarily separated from the main body 100, two coupling hooks 201 are provided on the edge of the lower surface of the upper end of the steamer 200, and when the coupling hooks 201 are inserted into the coupling grooves 101 formed on the upper surface of the main body 100, the coupling hooks 201 are fixed by hook coupling so that the steamer 200 may be fixed to the main body 100.

In this case, a push button is provided on the upper outer circumferential surface of the steamer 200 to push out the coupling hook 201, and when the coupling hook 201 is pushed out by the operation of the push button, it is spaced apart from the coupling groove 101 so that the steamer 200 may be separated.

Further, it is preferable that a spring is formed inside the push button so as to return the coupling hook 201 to a position where it is fastened to the coupling groove 101, so that the steamer 200 may be separated only while the push button is kept pressed.

In order to supply power to the steamer 200 in a state in which the main body 100 and the steamer 200 are coupled, a first connector 190 is formed at an upper center of the main body 100, and a second connector 270 formed to be in contact with the first connector 190 is provided at a center of a lower surface of the steamer 200.

The first connector 190 and the second connector 270 are formed to receive DC power by contact. To this end, the first connector 190 and the second connector 270 are formed with a plurality of ring-shaped power terminals at regular intervals, and the first connector 190 and the second connector 270 are formed at different intervals from each other so that each power terminal may be overlapped and contacted when coupled.

By the contact between the first connector 190 and the second connector 270, the steamer 200 may be freely separated from the main body 100, and when coupled, may receive power from the main body 100 to heat water and generate steam.

Further, the first connector 190 and the second connector 270 are provided with separate terminals for data processing, so that they may transmit a control signal for controlling the steamer 200 based on information set in the main body 100, and may also transmit information on steam and temperature information measured in the steamer 200 to the main body 100.

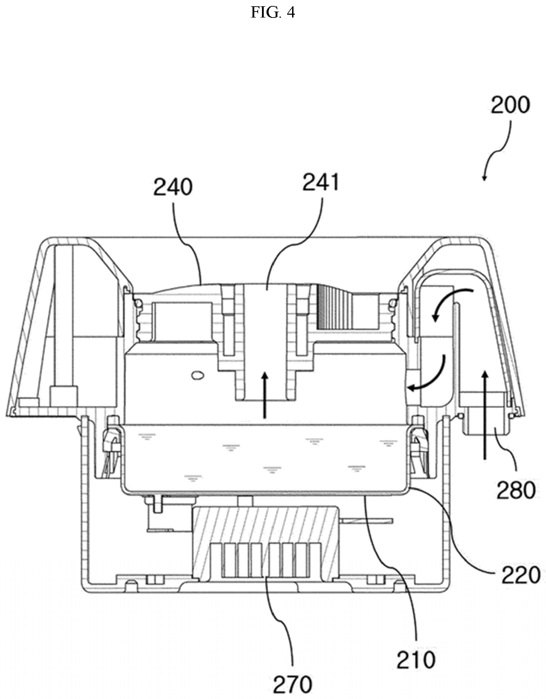

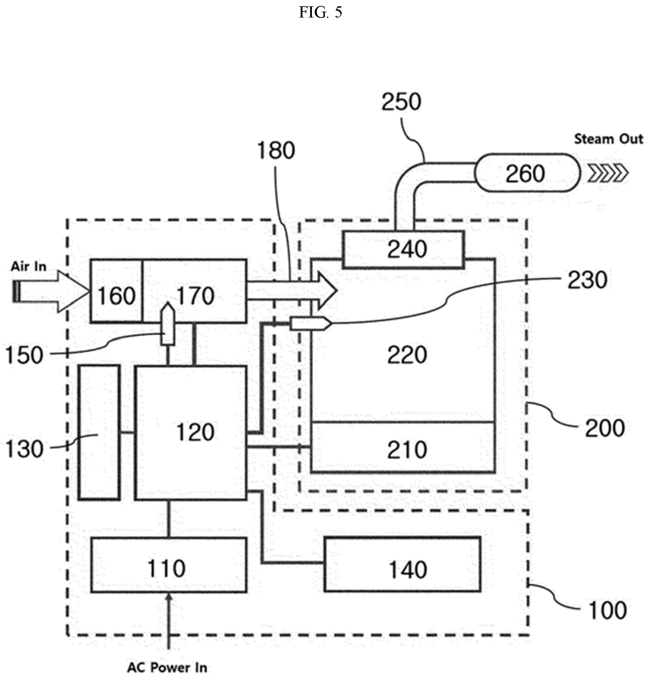

FIG. 4 is a cross-sectional view illustrating an internal structure of the steamer of the wet thermotherapy device capable of improving runny nose and nasal congestion symptoms using control of temperature, flow rate, and pressure of steam according to the disclosure, and FIG. 5 is a configuration view illustrating a coupling and operation structure of the wet thermotherapy device capable of improving runny nose and nasal congestion symptoms using control of temperature, flow rate, and pressure of steam according to the disclosure.

As illustrated in FIGS. 4 and 5, the main body 100 of the wet thermotherapy device capable of improving runny nose and nasal congestion symptoms using control of temperature, flow rate, and pressure of steam according to the disclosure includes a blower fan 170 that forms external air with a set flow rate and pressure by rotation and supplies the external air to the steamer 200, a filter 160 formed on a front surface of the blower fan 170 to collect and purify foreign matter, germs, and viruses mixed in the external air, an outside air temperature sensor 150 formed on the front surface of the blower fan 170 to measure a temperature of the external air being sucked in so as to adjust in advance the temperature of steam generated in the steamer 200, and a supply flow path 180 for supplying the external air supplied by the blower fan 170 into the steamer 200, wherein the blower fan 170 maintains the flow rate of the external air at 1.5 to 2.5 CFM and the pressure at 75 to 85 mmH2O.

Further, the steamer 200 includes a heating container 220 having an open upper portion to accommodate water and made of a stainless steel material, a heater 210 formed on a lower surface of the heating container 220 to receive power from the main body 100 and heat the heating container 220 to generate steam, an inlet flow path 280 formed on one upper side of the heating container 220 to allow external air supplied from the main body 100 to flow into an upper portion of the heating container 220, and an inside air temperature sensor 230 formed on the other upper side of the heating container 220 to measure a temperature of steam in a state in which external air is introduced and to control the temperature of the heater 210, wherein the heater 210 maintains the temperature of steam discharged through the nozzle 260 at 42 to 44 degrees Celsius.

A power supply unit 110 that receives AC power from the outside, converts it into DC power, and supplies it is formed inside the main body 100, and the power supply unit 110 is formed to supply driving power by a control unit 120 formed inside the main body 100.

The control unit 120 is formed to control all operations of the device by a firmware program built in a microcomputer, and is formed to display information on operations through the display 130 formed on the outer surface of the main body 100 and to allow a user to input or control a desired setting value.

Further, the control unit 120 is formed with a communication module 140 for transmitting/receiving data to/from an externally formed device through wireless communication or for being remotely controlled, and may be linked with a smartphone 300 based on the communication module 140 so as to be controlled by the smartphone 300 or to recommend a recommended treatment mode based on user information stored in the smartphone 300.

Since the main body 100 should discharge steam at a constant pressure and flow rate through the nozzle 260, it is formed to suck in external air and supply it into the steamer 200 while maintaining a constant pressure and flow rate.

To this end, a vent hole is provided on the outer surface of the main body 100 to suck in external air, and a filter 160 for collecting foreign matter contained in the external air or purifying germs and viruses is formed inside the vent hole.

In this case, it is preferable to use a HEPA filter 160 capable of collecting even fine dust and ultrafine dust mixed in the external air as the filter 160, thereby preventing inflammation from being aggravated when steam is sprayed inside the nasal cavity due to foreign matter mixed in the air.

The blower fan 170 is positioned on the rear surface of the filter 160, receives power by the power supply unit 110, and rotates by the control unit 120 to suck external air into the main body 100.

By the operation of the blower fan 170, external air is drawn into the main body 100 through the filter 160, and may be drawn into the steamer 200 through the supply flow path 180 in a state in which the flow rate and pressure are adjusted by the blower fan 170.

In this case, it is preferable that the blower fan 170 supplies the flow rate of external air at 2.0 to 2.5 CFM and the pressure at 80 to 85 mmH2O, and the flow rate of steam and external air sprayed from the nozzle 260 should be maintained at a minimum of 2.0 CFM and the pressure at 80 mmH2O or more so that the steam may reach the inside of the nasal cavity.

The external air supplied by the blower fan 170 enters the steamer 200, is mixed with steam generated inside the steamer 200, and is then sprayed through the nozzle 260 into the user's nasal cavity.

Further, the outside air temperature sensor 150 is formed between the filter 160 and the blower fan 170 so as to measure the temperature of external air, and the outside air temperature sensor 150 predicts the temperature when external air is mixed with the steam generated inside the steamer 200, and may be used as data for the control unit 120 to adjust the temperature of the heater 210 formed in the steamer 200.

That is, when external air is in a cold state, the external air supplied into the steamer 200 by the blower fan 170 is mixed with steam, and the temperature of steam decreases. In order to maintain the temperature of steam for rhinitis treatment constant, when the temperature of external air is low, the power of the heater 210 is increased to generate higher temperature steam.

Conversely, when external air is in a warm state, the temperature change of steam is expected to be small when external air is supplied into the steamer 200, so the power of the heater 210 may be lowered.

Therefore, the temperature inside the steamer 200 may be appropriately adjusted by measuring the temperature of external air in real time, and based on this, the temperature drop phenomenon that occurs when steam is mixed with external air may be suppressed.

The steamer 200 has the inlet flow path 280 formed on the lower surface of the upper end thereof, and the inlet flow path 280 is inserted into the supply flow path 180 to spray the air discharged from the blower fan 170 from one upper side of the heating container 220 and mix it with steam.

In this case, the heating container 220 made of a stainless steel material is built in the upper center of the steamer 200, and the heater 210 is formed on a lower surface of the heating container 220 so as to transfer heat to the lower surface of the heating container 220.

In this case, it is preferable that the heater 210 is formed integrally with the heating container 220 as a thick film heater 210 (Thick Film Heater) having a high power density and fast heat transfer, and the heater 210 is connected to the second connector 270 and may receive power when the second connector 270 is coupled to the first connector 190 formed on the main body 100.

The heater 210 is formed to be supplied with power and controlled by the main body 100, and may heat the heating container 220 to heat water accommodated in the heating container 220 to generate steam.

Steam generated as water is heated is prevented from leaking to the outside by the cover 240 coupled to the upper center of the steamer 200, and may be discharged only through the nozzle 260 along the discharge hole 241 formed in the cover 240.

Since the steam should be able to enter the inside of the nasal cavity for rhinitis treatment, the external air supplied from the main body 100 is formed at a set flow rate and pressure and then may enter the steamer 200 through the inlet flow path 280.

The external air entering the steamer 200 is brought into contact with steam to be mixed, and may be discharged to the outside through the tube 250 and the injection holes 261 formed in the nozzle 260 via the discharge hole 241 of the cover 240, which is the only passage.

In this case, it is preferable that the temperature of the steam discharged through the nozzle 260 is maintained at 40 to 45 degrees Celsius for rhinitis treatment, and when the temperature of steam is less than 40 degrees Celsius, heat loss occurs while moving through the tube 250, so that the steam becomes a low temperature state when entering the nasal cavity due to the temperature drop, and the therapeutic effect is reduced.

Further, when the temperature of steam exceeds 45 degrees Celsius, since the temperature of the steam discharged through the nozzle 260 is high, a low-temperature burn may occur inside the nasal cavity.

As a result, considering the heat loss that occurs while moving through the tube 250, it is preferable to maintain the temperature of steam generated in the steamer 200 at 40 to 45 degrees Celsius.

In order to measure the temperature of steam, the inside air temperature sensor 230 is formed on the other upper side of the heating container 220, and the inside air temperature sensor 230 measures the temperature of steam in real time so as to measure the temperature that changes due to mixing when external air enters the steamer 200.

In this case, the inside air temperature sensor 230 measures the temperature of steam generated by initially boiling water in the heating container 220, and may detect the steam and internal air temperatures at the upper portion of the heating container 220 and transmit the temperature information to the control unit 120.

Until the water boils in the heating container 220, the heater 210 is continuously heated, and when steam generated by boiling water through the inside air temperature sensor 230 is generated and reaches 60 degrees Celsius, the blower fan 170 is operated, and the power of the heater 210 may be controlled at an interval based on the outside air temperature sensor 150.

That is, the temperature of the heated steam is controlled to 40 to 45 degrees Celsius by external air and may be discharged through the nozzle 260.

The interval condition proceeds with N seconds Off / N seconds On, and is continuously repeated until the operation ends, and the interval control condition of the heater 210 varies in real time according to the temperature of external air supplied through the blower fan 170.

If the temperature of external air decreases, the temperature of steam is lowered by the inside air temperature sensor 230, so the power of the heater 210 is increased to raise the temperature of steam, and in order to suppress such a temperature change of steam, the heater 210 may be operated in advance based on the outside air temperature sensor 150.

As a result, the steam may be sprayed into the nasal cavity of the human body by the temperature, pressure, and flow rate of the steam, so that harmful germs and viruses in the nasal cavity may be eliminated, and the inflammation of the paranasal sinus may be treated to treat allergic rhinitis, nasal congestion, and sinusitis, and respiratory diseases such as colds may be prevented.

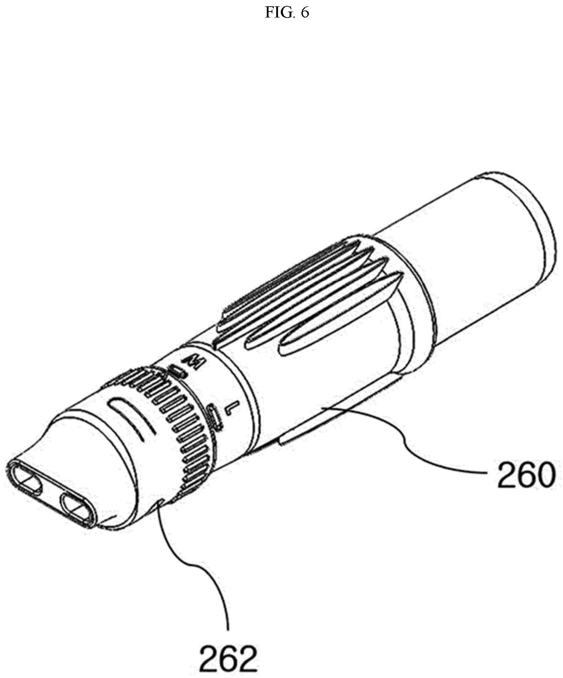

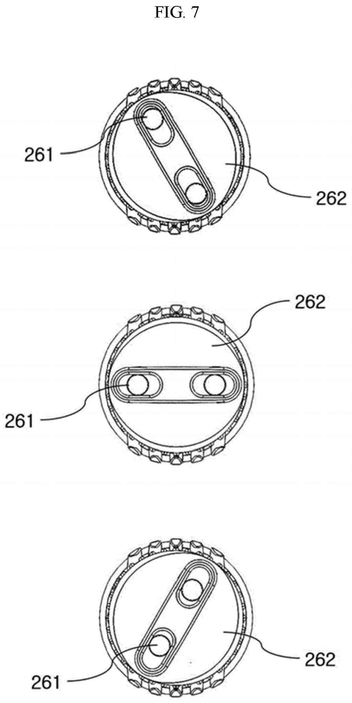

FIG. 6 is a perspective view illustrating a variable nozzle 260 of the wet thermotherapy device capable of improving runny nose and nasal congestion symptoms using control of temperature, flow rate, and pressure of steam according to the disclosure, and FIG. 7 is a front view illustrating a state in which a position where steam is discharged is varied by an adjustment cap 262 in the nozzle 260 of the wet thermotherapy device capable of improving runny nose and nasal congestion symptoms using control of temperature, flow rate, and pressure of steam according to the disclosure.

As illustrated in FIGS. 6 and 7, the nozzle 260 of the wet thermotherapy device capable of improving runny nose and nasal congestion symptoms using control of temperature, flow rate, and pressure of steam according to the disclosure further includes the adjustment cap 262 formed on an outer surface thereof and formed to adjust an interval of the plurality of injection holes 261 to a user's nostril position by rotation.

The adjustment cap 262 is formed at the other end of the nozzle 260 and is used to narrow or widen the interval of the injection holes 261 through which steam is sprayed so as to adjust to the user's nostril position.

To this end, the adjustment cap 262 is formed to be rotatable at a certain angle, and when rotated clockwise, the interval between the injection holes 261 is widened, and when rotated counterclockwise, the interval of the injection holes 261 is narrowed.

Therefore, since the interval of the injection holes 261 may be adjusted to the user's nostril interval, the steam may be sprayed to the exact center of the nostril and enter the inside of the nasal cavity.

Further, S, M, and L units are described at the other end of the nozzle 260 so that the interval of the injection holes 261 may be visually identified through the adjustment cap 262, and it is preferable that the adjustment cap 262 is formed to automatically stop when reaching each unit.

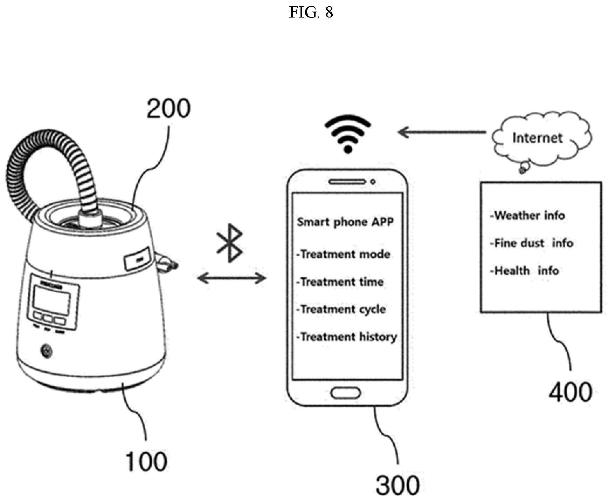

FIG. 8 is an exemplary view illustrating a state in which the wet thermotherapy device capable of improving runny nose and nasal congestion symptoms using control of temperature, flow rate, and pressure of steam according to the disclosure is linked with a smartphone.

As illustrated in FIG. 8, the main body 100 of the wet thermotherapy device capable of improving runny nose and nasal congestion symptoms using control of temperature, flow rate, and pressure of steam according to the disclosure is formed to wirelessly communicate with the smartphone 300 in which information on a user's treatment mode, treatment time, treatment cycle, and treatment history is input, such that the operation time of the steamer 200 and the temperature, flow rate, and pressure of steam are automatically adjusted by the smartphone 300.

The communication module 140 is formed in the main body 100 so as to be linked with the user's smartphone 300 through wireless communication, and the setting values of the control unit 120 may be changed or the operating state may be controlled through the smartphone 300.

In this case, it is preferable that an application for controlling and operating the rhinitis treatment device is installed on the smartphone 300, and the application may receive in real time the operating state and treatment history data of the rhinitis treatment device based on the communication module 140.

The smartphone 300 is formed to transmit/receive data through wireless communication with the communication module 140 using Bluetooth, and the treatment mode, treatment time, and treatment cycle of the rhinitis treatment device may be set and operated through the application of the smartphone 300.

Further, even if a plurality of users use the rhinitis treatment device by using the treatment history that has previously used the rhinitis treatment device, the treatment mode, treatment time, and treatment cycle of the rhinitis treatment device may be automatically adjusted using the user's own treatment history based on the smartphone 300.

In detail, since the temperature, flow rate, and pressure of steam may be automatically adjusted based on the user's treatment history input to the smartphone 300, the user may easily use the rhinitis treatment device without separately manipulating it through the main body 100.

Further, the main body 100 receives at least one of weather information, fine dust information, health information, disease information, and prescription information of a current region through wireless communication and recommends a recommended treatment mode according to an environmental condition or a user condition.

The smartphone 300 may receive external environment information of the Internet 400 using Wi-Fi or a mobile communication network and transmit it to the control unit 120, and the external environment information may receive at least one of weather information, fine dust information, health information, disease information, and prescription information of the current region where the user is located.

Based on the external environment information as above, treatment conditions are recommended to the user, operating conditions are optimized, and the user's treatment history is managed so that customized treatment according to the user's situation may be achieved, thereby maximizing the rhinitis treatment effect.

Further, the health information and disease information may be used to take measures to prevent respiratory infectious diseases by shortening the rhinitis treatment cycle and lengthening the treatment time using steam when the degree of infection is high based on information related to respiratory diseases.

In the case of prescription information, it includes the disease name diagnosed by a doctor and the medication taken, and the temperature, pressure, and flow rate of steam may be adjusted to perform effective treatment based on the relevant information.

That is, when the nose is blocked, it is difficult for steam to reach the inside of the nasal cavity, so the temperature, pressure, and flow rate of steam are increased, and when the rhinitis treatment device is used for prevention, the temperature, pressure, and flow rate of steam are decreased to prevent damage from occurring inside the nasal cavity.

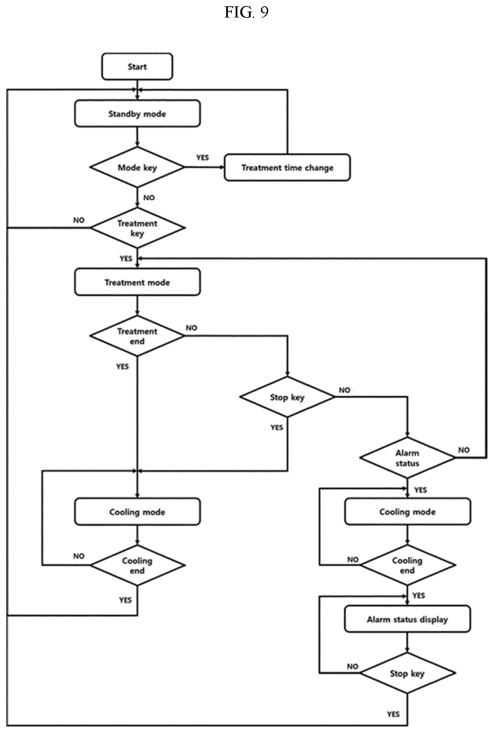

FIG. 9 is a flowchart illustrating a control process logic of the wet thermotherapy device capable of improving runny nose and nasal congestion symptoms using control of temperature, flow rate, and pressure of steam according to the disclosure.

As illustrated in FIG. 9, looking at the process of the wet thermotherapy device capable of improving runny nose and nasal congestion symptoms using control of temperature, flow rate, and pressure of steam according to the disclosure, an initial user opens the cover 240 of the steamer 200, injects an appropriate amount of water into the container using a measuring cup, closes the cover 240, couples the external tube 250 and the nozzle 260, and prepares to operate the main body 100.

Thereafter, when the power switch of the main body 100 is turned on to apply power, the control process of the control unit 120 starts and enters a standby mode.

The user may select a treatment time-mode of 20 minutes, 15 minutes, 10 minutes, or 5 minutes through the display 130 or the smartphone 300, and when the user presses the start button, it is changed to the treatment mode, voltage is supplied to the heater 210, and power is also applied to the blower, so that the treatment mode proceeds.

In this case, it is possible to determine whether an alarm state is in place by monitoring whether the corresponding components are operating correctly and the external air temperature and the internal temperature of the steamer 200, and when malfunction is detected, the power of the heater 210 is cut off, the cooling mode is switched, and then the treatment mode ends.

Further, when the user presses the stop button provided on the display 130 or the smartphone 300 as needed during the treatment mode, it may be terminated immediately after switching to the cooling mode.

When the alarm state is normal, when the treatment mode starts, power is supplied to the heater 210 and water in the container is heated. When the water boils, the inside air temperature sensor 230 inside the steamer 200 detects the internal temperature and, as a signal for the user to start treatment, displays an icon indicating that treatment is in progress on the display 130 or the smartphone 300 and also provides sound information.

In the treatment mode, the heater 210 is heated in an interval form by the logic implemented based on the information of the outside air temperature sensor 150 and the inside air temperature sensor 230 to control the temperature of the steam at the outlet of the nozzle 260 to be maintained at 40 to 45 degrees Celsius.

In this case, the user places the outlet portion of the nozzle 260 at a point spaced about 2 to 3 cm from the nostril and performs thermal treatment so that the steam sprayed from the nozzle 260 is well introduced into the nostril during the treatment mode time.

Thereafter, after the treatment mode proceeds normally for the set time, the process is automatically switched to the cooling mode, and the operation of the device is automatically terminated after a certain time.

If water is insufficient inside the heating container 220 even during the treatment mode, the temperature change of the inside air temperature sensor 230 occurs, so this may be detected to notify the user with an alarm and automatically terminate the treatment.

As described above, according to the wet thermotherapy device capable of improving runny nose and nasal congestion symptoms using control of temperature, flow rate, and pressure of steam according to the disclosure, in order to maximize the effect of the local thermal treatment for rhinitis, the temperature of steam is maintained constant and the flow rate and pressure of steam are maintained constant so that the steam may be delivered to the inside of the nasal cavity, it is harmless to the human body even when inhaled by a user or used for a long time by using only saturated steam generated by purely boiling water without using drugs or other gases and light rays, harmful germs may be removed without unnecessary irritation to the affected area, runny nose symptoms and nasal congestion symptoms may be improved, inflammation may be alleviated, and respiratory diseases may be prevented by spraying steam without physical contact with the affected area for treatment, and customized treatment according to the user's situation is possible by recommending the user's treatment condition based on external environment information and optimizing operating conditions.

As described above, the disclosure has been described with reference to preferred embodiments, but those skilled in the art to which the disclosure pertains may variously modify or transform the disclosure within a range not departing from the technical idea and scope described in the claims of the disclosure. Therefore, the scope of the disclosure should be interpreted by the claims described to include examples of such various modifications.

## Claims

1. A wet thermotherapy device capable of improving runny nose and nasal congestion symptoms using control of temperature, flow rate, and pressure of steam, comprising:
a steamer having an open upper portion to accommodate water therein and heat the water to form steam;
a cover provided with a discharge hole in a center thereof to seal the open upper portion of the steamer so as to prevent water from leaking and to discharge steam to an outside;
a nozzle in which a tube is formed at one end such that the tube is coupled to the discharge hole to receive steam, and a plurality of injection holes are provided at the other end so that steam may be sprayed to each nostril; and
a main body which is formed such that the steamer may be mounted on an upper portion thereof and which controls an operation of the steamer and supplies external air into the steamer so as to maintain a temperature, pressure, and flow rate of steam sprayed from the nozzle constant.

2. The wet thermotherapy device of claim 1,
wherein the main body comprises:
a blower fan that forms external air with a set flow rate and pressure by rotation and supplies the external air to the steamer;
a filter formed on a front surface of the blower fan to collect and purify foreign matter, germs, and viruses mixed in the external air;
an outside air temperature sensor formed on the front surface of the blower fan to measure a temperature of the external air being sucked in so as to adjust in advance a temperature of steam generated in the steamer; and
a supply flow path for supplying the external air supplied by the blower fan into the steamer,
wherein the blower fan maintains the flow rate of the external air at 2.0 to 2.5 CFM and the pressure at 80 to 85 mmH2O.

3. The wet thermotherapy device of claim 1,
wherein the steamer comprises:
a heating container having an open upper portion to accommodate water and made of a stainless steel material;
a heater formed on a lower surface of the heating container to receive power from the main body and heat the heating container to generate steam;
an inlet flow path formed on one upper side of the heating container to allow external air supplied from the main body to flow into an upper portion of the heating container; and
an inside air temperature sensor formed on the other upper side of the heating container to measure a temperature of steam in a state in which external air is introduced and to control a temperature of the heater,
and wherein the heater maintains a temperature of steam discharged through the nozzle at 40 to 45 degrees Celsius.

4. The wet thermotherapy device of claim 1,
wherein the main body is formed to wirelessly communicate with a smartphone in which information on a user's treatment mode, treatment time, treatment cycle, and treatment history is input, such that an operation time of the steamer and a temperature, flow rate, and pressure of steam are automatically adjusted by the smartphone.

5. The wet thermotherapy device of claim 1,
wherein the main body receives at least one of weather information, fine dust information, health information, disease information, and prescription information of a current region through wireless communication and recommends a recommended treatment mode according to an environmental condition or a user condition.

6. The wet thermotherapy device of claim 1,
wherein the nozzle further comprises an adjustment cap formed on an outer surface thereof and formed to adjust an interval of the plurality of injection holes to a user's nostril position by rotation.
